# EUROPEAN PATENT APPLICATION

(11) **EP 2 009 003 A1**
(43) Date of publication of application: **31.12.2008**
(21) Application number: 08252212.9
(22) Date of filing: 27.06.2008
(51) Int. Cl.: C07D 239/42, C07D 239/48, C07D 473/28

(54) **Production method of diaminopyrimidine compounds**

(30) Priority: 27.06.2007 JP 2007168879
(71) Applicant: Ajinomoto Co., Inc., Tokyo 104-8315 (JP)
(72) Inventor: Oka, Sachiko c/o Ajinomoto Co.,Inc, Kanagawa 210-8681 (JP); Takahashi, Daisuki c/o Ajinomoto Co.,,Inc, Mie 510-0885 (JP); Izawa, Kunisuke c/o Ajinomoto Co., Inc, Kawasaki-shi, Kanagawa 210-8681 (JP)
(74) Representative: Nicholls, Kathryn Margaret

(57) **Abstract**

The present invention relates to a novel production method of a diaminopyrimidine compound useful as an intermediate for various compounds having a pharmacological activity, a novel intermediate useful for producing said compound, and a production method of the intermediate.

The present invention provides a novel 5-aminopyrimidine compound represented by the following formula (2) and a novel 4,5-diaminopyrimidine compound represented by the formula (3), as well as production methods of the compounds of the following formulas (2) to (6). 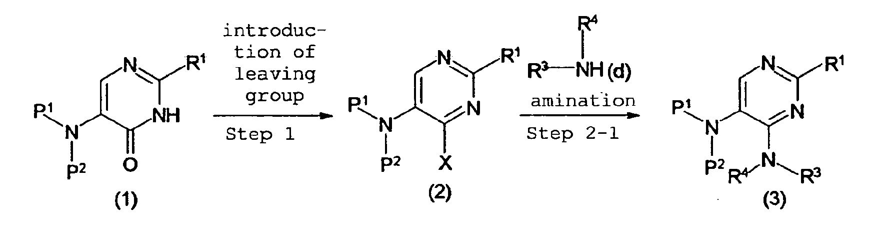 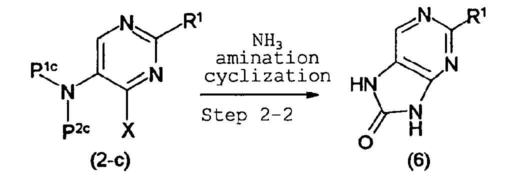 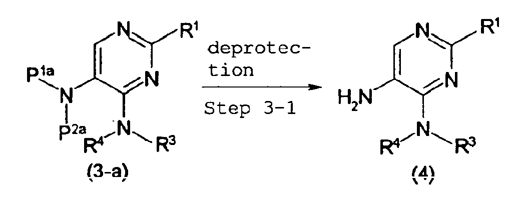 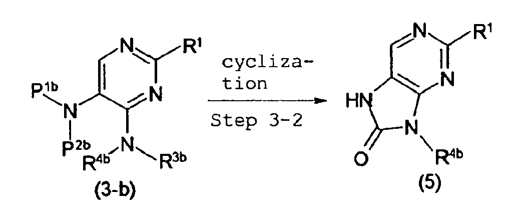 wherein each symbol is as defined in the specification.

## Description

### Technical Field

The present invention relates to a novel production method of a diaminopyrimidine compound useful as an intermediate for various compounds having a pharmacological activity, a novel intermediate useful for producing said compound, and a production method of the intermediate.

### Background Art

A 4,5-diaminopyrimidine compound of the following formula and a derivative thereof, i.e., 8-oxodihydropurine compound, are useful as intermediates for compounds having various pharmacological activities, such as an antiobesity drug, an antidepressant and the like (WO99/28320, WO2006/001266).

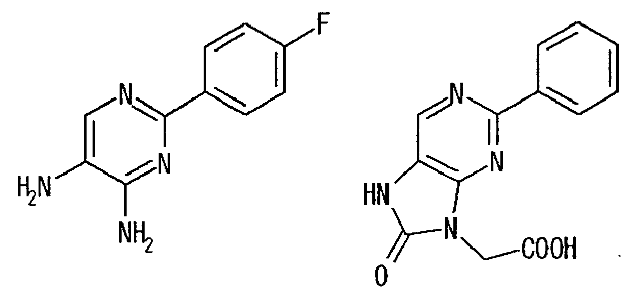

As the production method of a 4,5-diaminopyrimidine compound, a method including reduction of a 4-nitro-5-aminopyrimidine compound is known (WO99/28320). However, since this method includes a step through a potentially explosive nitro compound, a more preferable method for industrial production is desired. As the production method of a 8-oxodihydropurine compound, a method including, after production of a 4,5-diaminopyrimidine compound by the aforementioned method, reacting the obtained compound with carbonyldiimidazole or diethyl carbonate, or reacting a 4-amino-5-carboxypyrimidine compound with an azide compound such as sodium azide, diphenylphosphoryl azide and the like is known (WO99/28320). However, the former method is associated with the problem caused by the use of a nitro compound as mentioned above, and in the latter method, the azide compound is explodable. Similarly, therefore, a more preferable method for industrial production is desired.

In Tetrahedron Letters, 1966, 37, 4517-4521 and H.W. van Meeteren, RECUEIL, 1968, 87, 1089-1098, it is reported that an attempt was made to treat 5-amino-4-chloro-2-phenylpyrimidine with potassium amide in an ammonia solution to convert same to a 4-amino derivative, but a skeletal rearrangement occurred to afford cyanoimidazole and the attempt failed.

Also, in H.W. van Meeteren, RECUEIL, 1968, 87, 1089-1098 and L. Maarchanl, Bull. Soc. Chim. Belg., 1960, 69, 177-193, it is reported that an attempt was made to treat 5-amino-4-oxopyrimidine or 5-amino-4-oxo-2-phenylpyrimidine with phosphorus oxychloride or phosphorus pentachloride to convert same to a 4-chloro derivative, but the attempt failed.

### Disclosure of the Invention

Accordingly, the problem to be solved by the present invention is provision of a method of efficiently producing a diaminopyrimidine compound of the below-mentioned formula (4), as well as a 8-oxodihydropurine compounds of the below-mentioned formulas (5) and (6), by industrially suitable methods, and further, an intermediate useful for the production, and a production method of the intermediate.

The present inventors have conducted intensive studies in an attempt to solve the aforementioned problems and found that when a 5-amino-4-oxopyrimidine compound of the below-mentioned formula (1), wherein the amino group at the 5-position is protected, is used as a staring compound, introduction (e.g., chlorination etc.) of a leaving group into the 4-position proceeds smoothly, and a 5-aminopyrimidine compound of the below-mentioned formula (2), which is represented by a 5-amino-4-chloropyrimidine compound, can be obtained in a good yield.

In addition, they have found that the 4-position is efficiently aminated and a 4,5-diaminopyrimidine compound of the below-mentioned formula (3) can be obtained by reacting a 5-aminopyrimidine compound of the formula (2) with ammonia or an amine compound.

Moreover, they have found that when the 5-aminopyrimidine compounds of the formula (2) wherein the amino group is protected with a urethane-type protecting group (5-aminopyrimidine compound of the below-mentioned formula (2-c)) is reacted with ammonia, a 8-oxodihydropurine compound of the below-mentioned formula (6) can be obtained, and that a 4,5-diaminopyrimidine compound of the formula (3) wherein the amino group is protected with a urethane-type protecting group, R³ is a hydrogen atom and R⁴ is not a hydrogen atom (4,5-diaminopyrimidine compound of the below-mentioned formula (3-b)) can be led to a 8-oxodihydropurine compound of the below-mentioned formula (5) under particular conditions. Based on these findings, the present inventors have completed the present invention.

WO2006/127584 discloses in Examples 40 and 41 that, as shown below, a 4,5-diaminopyrimidine compound is produced via chlorination and amination steps of a 5-amino-4-oxopyrimidine compound having a diethylamino group at the 2-position, wherein the amino group at the 5-position is acylated or alkylated. Although the yield of the chlorination step in the reaction was 68%, when the present inventors tried chlorination of a compound having a dimethylamino group at the 2-position, wherein the amino group at the 5-position is not alkylated (acylation alone), the yield was markedly low (Reference Example 4). The yield of the subsequent amination step was 3.8%, and therefore, it is difficult to consider that an effective method is disclosed.

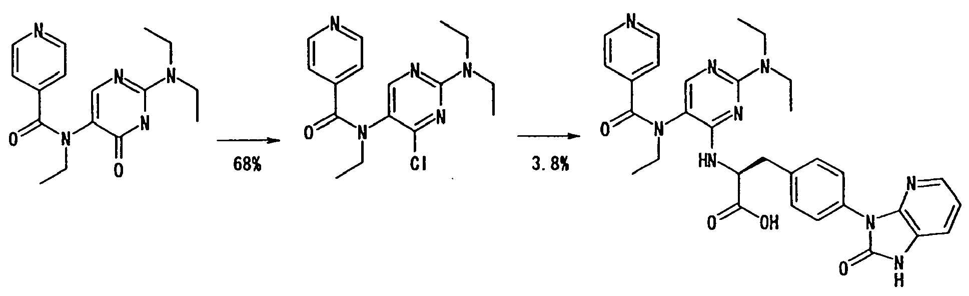

Accordingly, the present invention relates to the following:
[1] A 5-aminopyrimidine compound represented by the formula (2): 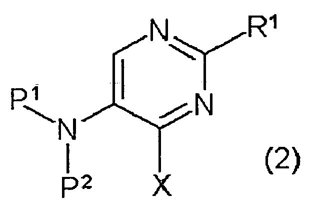 wherein
   X is a leaving group,
   P¹ is a hydrogen atom or a benzyl group optionally having substituent(s),
   P² is a urethane-type protecting group or an amide-type protecting group when P¹ is a hydrogen atom, or a benzyl group optionally having substituent(s) when P¹ is a benzyl group optionally having substituent(s), and
   R¹ is a hydrogen atom, an alkyl group optionally having substituent(s), an aryl group optionally having substituent(s), an aralkyl group optionally having substituent(s), or a group represented by the formula (a) or the formula (b):

      -0-R ² (a)

      -S-R² (b)

      wherein R² is an alkyl group optionally having substituent(s), an aryl group optionally having substituent(s) or an aralkyl group optionally having substituent(s),
   or a salt thereof (hereinafter sometimes to be generically referred to as compound (2)).
[2] A 4,5-diaminopyrimidine compound represented by the formula (3): 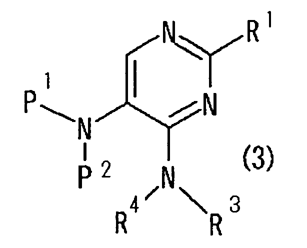 wherein
   P¹ is a hydrogen atom or a benzyl group optionally having substituent(s),
   P² is a urethane-type protecting group or an amide-type protecting group when P¹ is a hydrogen atom, or a benzyl group optionally having substituent(s) when P¹ is a benzyl group optionally having substituent(s),
   R¹ is a hydrogen atom, an alkyl group optionally having substituent(s), an aryl group optionally having substituent(s), an aralkyl group optionally having substituent(s), or a group represented by the formula (a) or the formula (b):

      -O-R² (a)

      -S-R² (b)

      wherein R² is an alkyl group optionally having substituent(s), an aryl group optionally having substituent(s) or an aralkyl group optionally having substituent(s),
   R³ is a hydrogen atom, an alkyl group optionally having substituent(s) or an aralkyl group optionally having substituent(s), and
   R⁴ is a hydrogen atom, an alkyl group optionally having substituent(s), an aralkyl group optionally having substituent(s), or a group represented by the formula (c): 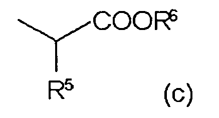 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
   provided that a compound wherein P¹ is a hydrogen atom, P² is a urethane-type protecting group and R³ and R⁴ are hydrogen atoms is excluded,
   or a salt thereof (hereinafter sometimes to be generically referred to as compound (3)).
[3] A production method of a 5-aminopyrimidine compound represented by the formula (2): 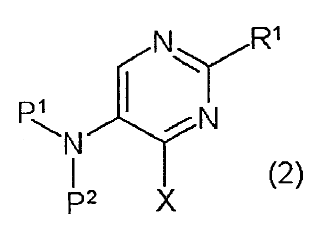 wherein
   X is a leaving group,
   P¹ is a hydrogen atom or a benzyl group optionally having substituent(s),
   P² is a urethane-type protecting group or an amide-type protecting group when P¹ is a hydrogen atom, or a benzyl group optionally having substituent(s) when P¹ is a benzyl group optionally having substituent(s), and
   R¹ is a hydrogen atom, an alkyl group optionally having substituent(s), an aryl group optionally having substituent(s), an aralkyl group optionally having substituent(s), or a group represented by the formula (a) or the formula (b):

      -0-R² (a)

      -S-R² (b)

      wherein R² is an alkyl group optionally having substituent(s), an aryl group optionally having substituent(s) or an aralkyl group optionally having substituent(s),
   or a salt thereof, which comprises converting the oxo group of a 5-amino-4-oxopyrimidine compound represented by the formula (1) : 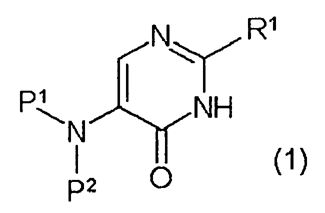 wherein each symbol is as defined above,
   or a salt thereof (hereinafter sometimes to be generically referred to as compound (1)), to a leaving group.
[4] A production method of a 4,5-diaminopyrimidine compound represented by the formula (3): 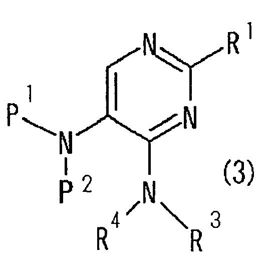 wherein
   P¹ is a hydrogen atom or a benzyl group optionally having substituent(s),
   P² is a urethane-type protecting group or an amide-type protecting group when P¹ is a hydrogen atom, or a benzyl group optionally having substituent(s) when P¹ is a benzyl group optionally having substituent(s),
   R¹ is a hydrogen atom, an alkyl group optionally having substituent(s), an aryl group optionally having substituent(s), an aralkyl group optionally having substituent(s), or a group represented by the formula (a) or the formula (b):

      -0-R² (a)

      -S-R² (b)

      wherein R² is an alkyl group optionally having substituent(s), an aryl group optionally having substituent(s) or an aralkyl group optionally having substituent(s),
   R³ is a hydrogen atom, an alkyl group optionally having substituent(s) or an aralkyl group optionally having substituent(s), and
   R⁴ is a hydrogen atom, an alkyl group optionally having substituent(s), an aralkyl group optionally having substituent(s), or a group represented by the formula (c): 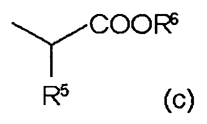 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
   or a salt thereof, which comprises reacting a 5-aminopyrimidine compound represented by the formula (2): 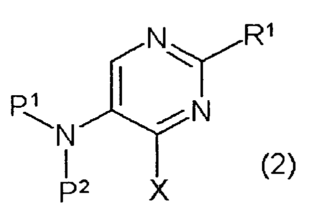 wherein X is a leaving group, and other symbols are as defined above,
   or a salt thereof, with an amine compound represented by the formula (d): 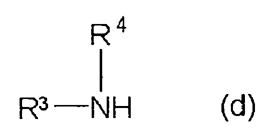 wherein each symbol is as defined above,
   or a salt thereof (hereinafter sometimes to be generically referred to as compound (d)),
   provided that when P¹ is a hydrogen atom and P² is a urethane-type protecting group, then both R³ and R⁴ should not be hydrogen atoms.
[5] A production method of a 8-oxodihydropurine compound represented by the formula (6): 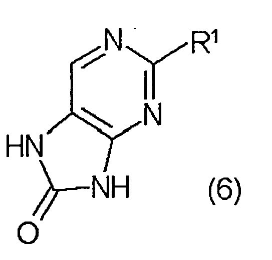 wherein
   R¹ is a hydrogen atom, an alkyl group optionally having substituent(s), an aryl group optionally having substituent(s), an aralkyl group optionally having substituent(s), or a group represented by the formula (a) or the formula (b):

      -0-R² (a)

      -S-R² (b)

      wherein R² is an alkyl group optionally having substituent(s), an aryl group optionally having substituent(s) or an aralkyl group optionally having substituent(s),
   or a salt thereof (hereinafter sometimes to be generically referred to as compound (6)), which comprises reacting a 5-aminopyrimidine compound represented by the formula (2-c): 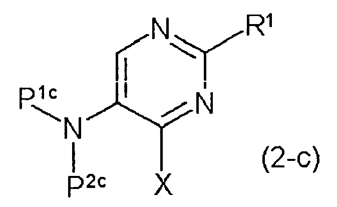 wherein
      X is a leaving group,
      P^{1c} is a hydrogen atom,
      P^{2c} is a urethane-type protecting group, and
      R¹ is as defined above,
   or a salt thereof (hereinafter sometimes to be generically referred to as compound (2-c)) with ammonia.
[6] A production method of a 4,5-diaminopyrimidine compound represented by the formula (4): 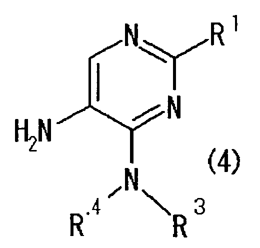 wherein
   R¹ is a hydrogen atom, an alkyl group optionally having substituent(s), an aryl group optionally having substituent(s), an aralkyl group optionally having substituent(s), or a group represented by the formula (a) or the formula (b):

      -0-R² (a)

      -S-R² (b)

      wherein R² is an alkyl group optionally having substituent(s), an aryl group optionally having substituent(s) or an aralkyl group optionally having substituent(s),
   R³ is a hydrogen atom, an alkyl group optionally having substituent(s) or an aralkyl group optionally having substituent(s), and
   R⁴ is a hydrogen atom, an alkyl group optionally having substituent(s), an aralkyl group optionally having substituent(s), or a group represented by the formula (c): 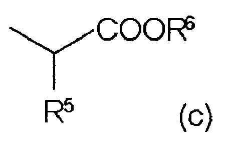 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
   or a salt thereof (hereinafter sometimes to be generically referred to as compound (4)), which comprises removing the amino-protecting group at the 5-position of a 4,5-diaminopyrimidine compound represented by the formula (3-a): 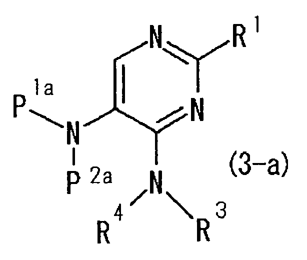 wherein
      P^{1a} is a hydrogen atom or a benzyl group optionally having substituent(s),
      P^{2a} is an amide-type protecting group when P^{1a} is a hydrogen atom, or a benzyl group optionally having substituent(s) when P^{1a} is a benzyl group optionally having substituent(s), and other symbols are as defined above,
   or a salt thereof (hereinafter sometimes to be generically referred to as compound (3-a)).
[7] A production method of a 8-oxodihydropurine compound represented by the formula (5): 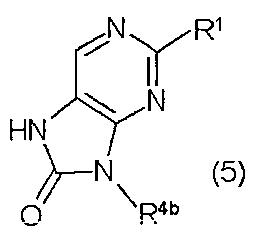 wherein
   R¹ is a hydrogen atom, an alkyl group optionally having substituent(s), an aryl group optionally having substituent(s), an aralkyl group optionally having substituent(s), or a group represented by the formula (a) or the formula (b):

      -0-R² (a)

      -S-R² (b)

      wherein R² is an alkyl group optionally having substituent(s), an aryl group optionally having substituent(s) or an aralkyl group optionally having substituent(s), and
   R^{4b} is an alkyl group optionally having substituent(s), an aralkyl group optionally having substituent(s), or a group represented by the formula (c): 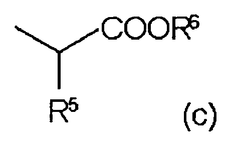 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
   or a salt thereof (hereinafter sometimes to be generically referred to as compound (5)), which comprises converting a 4,5-diaminopyrimidine compound represented by the formula (3-b) : 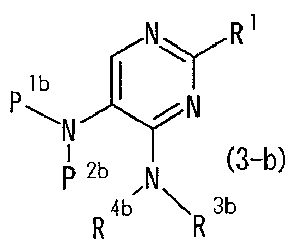 wherein
      P^{1b} is a hydrogen atom,
      P^{2b} is a urethane-type protecting group,
      R^{3b} is a hydrogen atom, and
      other symbols are as defined above,
   or a salt thereof (hereinafter sometimes to be generically referred to as compound (3-b)) to a purine derivative.

According to the present invention, a method of efficiently producing a 4,5-diaminopyrimidine compound and a derivative thereof, i.e., a 8-oxodihydropurine compound, which are useful as intermediates for a compound having various pharmacological activities, such as an antiobesity drug, an antidepressant and the like, by an industrially suitable method can be provided.

The definitions of the symbols and terms used in the present specification are explained in the following.

Examples of the "leaving group" in the present specification include a halogen atom, an optionally substituted alkylsulfonyloxy group, an optionally substituted arylsulfonyloxy group and the like.

The "halogen atom" in the present specification means a fluorine atom, a chlorine atom, a bromine atom or an iodine atom.

The "alkyl group" in the present specification means a linear or branched chain alkyl group having 1 to 20, preferably 1 to 10, more preferably 1 to 6, carbon atoms. Specific examples include methyl, ethyl, propyl, isopropyl, butyl, isobutyl, sec-butyl, tert-butyl, pentyl, hexyl, heptyl, octyl, nonyl, decyl and the like.

Examples of the substituent of the "alkyl group optionally having substituent(s)" in the present specification include a halogen atom, a C₁₋₆ alkoxy group, a hydroxy group, a nitro group and the like. The number and substitutable position of the substituents are not particularly limited. Specific examples of the "alkyl group optionally having substituent(s)" include fluoromethyl, trifluoromethyl, chloromethyl, methoxymethyl, hydroxymethyl and the like.

The "alkyl group optionally having substituent(s)" is preferably a C₁₋₁₀ alkyl group, particularly preferably a C₁₋₆ alkyl group.

The "aryl group" in the present specification means an aryl group having 6 to 20, preferably 6 to 14, more preferably 6 to 10, carbon atoms. Specific examples include phenyl, 1-naphthyl, 2-naphthyl and the like.

Examples of the substituent of the "aryl group optionally having substituent(s)" in the present specification include a halogen atom, a C₁₋₆ alkyl group, a C₁₋₆ alkoxy group, a hydroxy group, a nitro group and the like. The number and substitutable position of the substituents are not particularly limited. Specific examples of the "aryl group optionally having substituent(s)" include phenyl, 1-naphthyl, 2-naphthyl, o-, m- or p-fluorophenyl, o-, m- or p-chlorophenyl, o-, m- or p-methylphenyl, o-, m- or p-methoxyphenyl, o-, m- or p-nitrophenyl, o-, m- or p-hydroxyphenyl and the like.

The "aryl group optionally having substituent(s)" is preferably a C₆₋₁₀ aryl group, particularly preferably phenyl.

The "aralkyl group" in the present specification means an aralkyl group wherein the aryl moiety is an aryl group having preferably 6 to 14, more preferably 6 to 10, carbon atoms, and the alkyl moiety is a linear or branched chain alkyl group having preferably 1 to 6, more preferably 1 to 3, carbon atoms. Specific examples include benzyl, 1-phenylethyl, 2-phenylethyl, 3-phenylpropyl, 1-naphthylmethyl, 2-naphthylmethyl and the like.

Examples of the substituent of the "aralkyl group optionally having substituent(s)" in the present specification include a halogen atom, a C₁₋₆ alkyl group, a C₁₋₆ alkoxy group, a hydroxy group, a nitro group and the like. The number and substitutable position of the substituents are not particularly limited. Specific examples of the "aralkyl group optionally having substituent(s)" include benzyl, 1-phenylethyl, 2-phenylethyl, 3-phenylpropyl, 1-naphthylmethyl, 2-naphthylmethyl, o-, m- or p-fluorobenzyl, o-, m- or p-chlorobenzyl, o-, m- or p-methylbenzyl, o-, m- or p-methoxybenzyl, o-, m- or p-nitrobenzyl, o-, m- or p-hydroxybenzyl and the like.

The "aralkyl group optionally having substituent(s)" is preferably a C₇₋₁₃ aralkyl group, particularly preferably benzyl.

Examples of the substituent of the "benzyl group optionally having substituent(s)" in the present specification include a halogen atom, a C₁₋₆ alkyl group, a C₁₋₆ alkoxy group, a hydroxy group, a nitro group and the like. The number and substitutable position of the substituents are not particularly limited. Specific examples of the "benzyl group optionally having substituent(s)" include benzyl, o-, m- or p-fluorobenzyl, o-, m- or p-chlorobenzyl, o-, m- or p-methylbenzyl, o-, m- or p-methoxybenzyl, o-, m- or p-nitrobenzyl, o-, m- or p-hydroxybenzyl and the like.

The "benzyl group optionally having substituent(s)" is preferably a benzyl group.

The "alkylsulfonyloxy group optionally having substituent(s)" in the present specification means a sulfonyloxy group substituted by the above-mentioned "alkyl group optionally having substituent(s)", and it is preferably a C₁₋₆ alkylsulfonyloxy group optionally substituted by halogen atom(s). Specific examples include methanesulfonyloxy, trifluoromethanesulfonyloxy and the like.

The "arylsulfonyloxy group optionally having substituent(s)" in the present specification means a sulfonyloxy group substituted by the above-mentioned "aryl group optionally having substituent(s)", and it is preferably a C₆₋₁₀ arylsulfonyloxy group optionally substituted by C₁₋₆ alkyl group(s). Specific examples include benzenesulfonyloxy, p-toluenesulfonyloxy and the like.

The "urethane-type protecting group" in the present specification is represented by, for example, P³-O-CO- wherein P³ is an alkyl group optionally having substituent(s) or an aralkyl group optionally having substituent(s).

The "urethane-type protecting group" is preferably a C₇₋₁₃ aralkyloxy-carbonyl group optionally substituted by a C₁₋₆ alkoxy group, a nitro group and the like (e.g., benzyloxycarbonyl, p-nitrobenzyloxycarbonyl); or a C₁₋₆ alkoxy-carbonyl group optionally substituted by halogen atom(s) (e.g., methoxycarbonyl, ethoxycarbonyl, tert-butoxycarbonyl, trichloroethoxycarbonyl), more preferably a C₇₋₁₃ aralkyloxy-carbonyl group (e.g., benzyloxycarbonyl) or a C₁₋₆ alkoxy-carbonyl group (e.g., methoxycarbonyl, ethoxycarbonyl, tert-butoxycarbonyl), particularly preferably benzyloxycarbonyl.

The "amide-type protecting group" in the present specification is represented by, for example, P⁴-CO- wherein P⁴ is an alkyl group optionally having substituent(s), an aryl group optionally having substituent(s) or an aralkyl group optionally having substituent(s).

The "amide-type protecting group" is preferably a C₁₋₆ alkyl-carbonyl group optionally substituted by halogen atom(s) (e.g., acetyl, trichloroacetyl, propionyl, pivaloyl); a C₆₋₁₀ aryl-carbonyl group optionally substituted by a C₁₋₆ alkoxy group, a nitro group and the like (e.g., benzoyl, p-methoxybenzoyl); or a C₇₋₁₃ aralkyl-carbonyl group optionally substituted by a C₁₋₆ alkoxy group, a nitro group and the like (e.g., phenylacetyl, phenylpropanoyl), more preferably a C₁₋₆ alkyl-carbonyl group (e.g., acetyl, propionyl, pivaloyl), a C₆₋₁₀ aryl-carbonyl group (e.g., benzoyl) or a C₇₋₁₃ aralkyl-carbonyl group (e.g., phenylacetyl, phenylpropanoyl), particularly preferably acetyl or phenylacetyl.

Examples of the C₁₋₆ alkyl group exemplified above as a substituent include methyl, ethyl, propyl, isopropyl, butyl, isobutyl, sec-butyl, tert-butyl, pentyl, hexyl and the like. Examples of the C₁₋₆ alkoxy group exemplified above as a substituent include methoxy, ethoxy, propoxy, isopropoxy, butoxy, isobutoxy, sec-butoxy, tert-butoxy, pentyloxy, hexyloxy and the like.

P¹ is preferably a hydrogen atom.

P² is preferably a urethane-type protecting group or an amide-type protecting group, more preferably a C₇₋₁₃ aralkyloxy-carbonyl group optionally substituted by a C₁₋₆ alkoxy group, a nitro group and the like; a C₁₋₆ alkoxy-carbonyl group optionally substituted by halogen atom(s); a C₁₋₆ alkyl-carbonyl group optionally substituted by a halogen atom and the like; a C₆₋₁₀ aryl-carbonyl group optionally substituted by a C₁₋₆ alkoxy group, a nitro group and the like; or a C₇₋₁₃ aralkyl-carbonyl group optionally substituted by a C₁₋₆ alkoxy group, a nitro group and the like, further more preferably a C₇₋₁₃ aralkyloxy-carbonyl group, a C₁₋₆ alkoxy-carbonyl group, a C₁₋₆ alkyl-carbonyl group, a C₆₋₁₀ aryl-carbonyl group or a C₇₋₁₃ aralkyl-carbonyl group, particularly preferably benzyloxycarbonyl, acetyl or phenylacetyl.

R¹ is preferably a C₆₋₁₀ aryl group, a C₁₋₆ alkoxy group or a C₁₋₆ alkylthio group, particularly preferably phenyl, methoxy or methylthio.

R³ is preferably a hydrogen atom, a C₁₋₆ alkyl group or a C₇-₁₃ aralkyl group, particularly preferably a hydrogen atom.

R⁴ is preferably a hydrogen atom, a C₁₋₆ alkyl group, a C₇₋₁₃ aralkyl group or a -CH₂COO-C₁₋₆ alkyl group, particularly preferably a hydrogen atom, methyl, benzyl or tert-butoxycarbonylmethyl.

P^{1a} is preferably a hydrogen atom.

P^{2a} is preferably an amide-type protecting group, more preferably a C₁₋₆ alkyl-carbonyl group optionally substituted by halogen atom(s); a C₆₋₁₀ aryl-carbonyl group optionally substituted by a C₁₋₆ alkoxy group, a nitro group and the like; or a C₇₋₁₃ aralkyl-carbonyl group optionally substituted by a C₁₋₆ alkoxy group, a nitro group and the like, further more preferably a C₁₋₆ alkyl-carbonyl group, a C₆₋₁₀ aryl-carbonyl group or a C₇₋₁₃ aralkyl-carbonyl group, particularly preferably acetyl or phenylacetyl.

P^{2b} is preferably a C₇₋₁₃ aralkyloxy-carbonyl group optionally substituted by a C₁₋₆ alkoxy group, a nitro group and the like; or a C₁₋₆ alkoxy-carbonyl group optionally substituted by halogen atom(s), more preferably a C₇₋₁₃ aralkyloxy-carbonyl group or a C₁₋₆ alkoxy-carbonyl group, particularly preferably benzyloxycarbonyl.

R^{4b} is preferably a C₁₋₆ alkyl group, a C₇₋₁₃ aralkyl group or a -CH₂COO-C₁₋₆ alkyl group, particularly preferably methyl, benzyl or tert-butoxycarbonylmethyl.

P^{2c} is preferably a C₇₋₁₃ aralkyloxy-carbonyl group optionally substituted by a C₁₋₆ alkoxy group, a nitro group and the like; or a C₁₋₆ alkoxy-carbonyl group optionally substituted by halogen atom(s), more preferably a C₇₋₁₃ aralkyloxy-carbonyl group or a C₁₋₆ alkoxy-carbonyl group, particularly preferably benzyloxycarbonyl.

Examples of the salt of compounds (1) to (6) and compound (d) include acid addition salts, for example, salts with an inorganic acid such as hydrochloric acid, hydrobromic acid, nitric acid, sulfuric acid, phosphoric acid and the like; salts with an organic acid such as formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like, and the like.

The production method of compounds (2) to (6) are explained in the following. Each compound can be produced, for example, according to the following scheme or a method analogous thereto.

Unless otherwise specified, the leaving group introduction reaction, amination reaction, cyclization reaction, deprotection and the like in the following steps are performed according to a method known per se, for example, Organic Synthesis, Comprehensive Organic Chemistry, Fieser and Fieser Reagent for Organic Synthesis, Protective Groups in Organic Synthesis and the like, or a method analogous thereto. 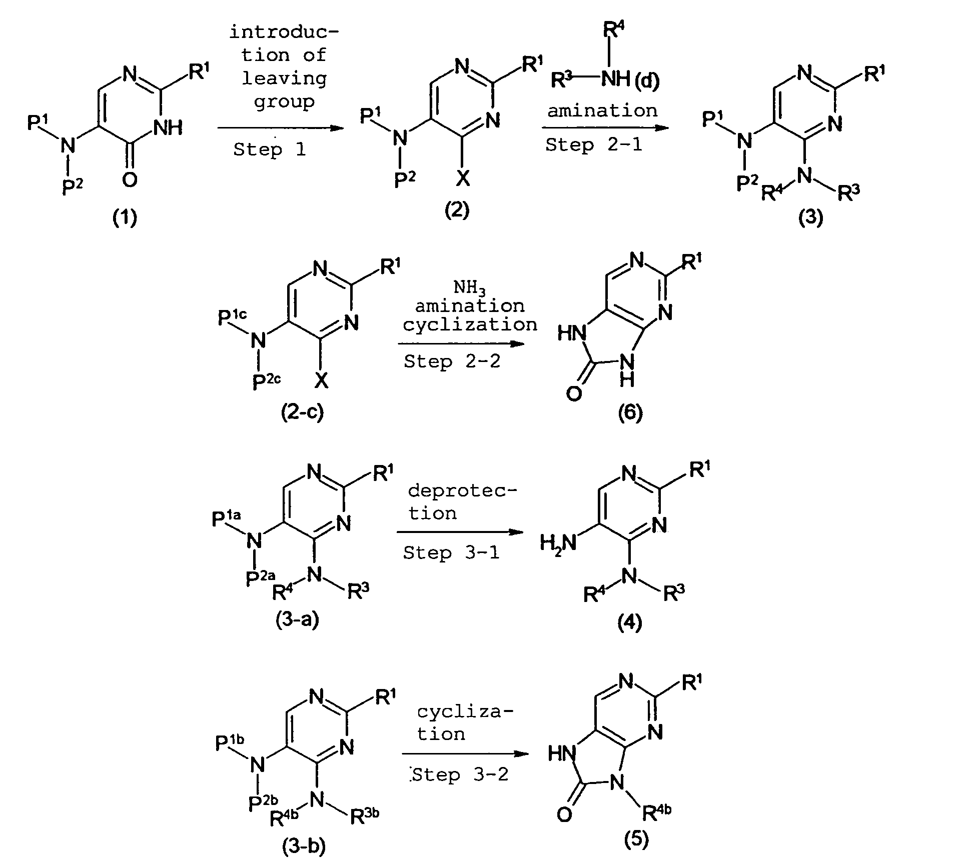 wherein each symbol is as defined above.

### Step 1: Introduction of leaving group (halogenation or sulfonylation)

In this step, compound (2) can be produced by converting the oxo group of compound (1) to a leaving group. The reaction is carried out by subjecting compound (1) to halogenation or sulfonylation.

The halogenation is carried out using a halogenating agent.

Examples of the halogenating agent include chlorinating agents such as phosphorus trichloride, phosphorus pentachloride, phosphorus oxychloride, thionyl chloride, sulfuryl chloride and the like; brominating agents such as phosphorus tribromide, phosphorus pentabromide and the like, and the like. Of these, a chlorinating agent is preferable, and phosphorus oxychloride is more preferable.

The amount of the halogenating agent to be used is generally 1 equivalent to a solvent amount, preferably 1 to 10 equivalents, more preferably 3 to 5 equivalents, relative to compound (1).

The halogenation is preferably carried out in the presence of a base and a quaternary ammonium compound.

Examples of the base include organic bases such as tertiary amines (e.g., triethylamine, diisopropylethylamine, dimethylaminopyridine, 1,8-diazabicyclo[5.4.0]undec-7-ene, 1,5-diazabicyclo[4.3.0]non-5-ene), aromatic amines (e.g., pyridine) and the like. Of these, a tertiary amine is preferable, and triethylamine is particularly preferable.

The amount of the base to be used is generally 0.2 to 10 equivalents, preferably 0.2 to 5 equivalents, more preferably 0.5 to 2 equivalents, relative to compound (1). When a halogenating agent such as phosphorus oxychloride and the like is used as a solvent, a base is not required.

Examples of the quaternary ammonium compound include tetraethylammonium chloride, tetraethylammonium bromide, tetra n-butylammonium chloride, tetra n-butyl ammonium bromide and the like. Of these, tetraethylammonium chloride is preferable.

The amount of the quaternary ammonium compound to be used is generally 1 to 10 equivalents, preferably 1 to 5 equivalents, more preferably 1.5 to 3 equivalents, relative to compound (1).

The sulfonylation is carried out using a sulfonylating agent.

Examples of the sulfonylating agent include a C₁₋₆ alkylsulfonyl chloride optionally substituted by halogen atom(s) (e.g., methanesulfonyl chloride, trifluoromethanesulfonyl chloride), a C₆₋₁₀ arylsulfonyl chloride optionally substituted by C₁₋₆ alkyl group(s) (e.g., benzenephenylsulfonyl chloride, p-toluenesulfonyl chloride) and the like. Of these, methanesulfonyl chloride, trifluoromethanesulfonyl chloride, benzenesulfonyl chloride and p-toluenesulfonyl chloride are preferable, and methanesulfonyl chloride is more preferable.

The amount of the sulfonylating agent to be used is generally 1 to 10 equivalents, preferably 1 to 5equivalents, more preferably 2 to 5 equivalents, relative to compound (1).

The sulfonylation reaction is preferably carried out in the presence of a base.

Examples of the base include inorganic bases such as alkali metal hydroxides (e.g., lithium hydroxide, sodium hydroxide, potassium hydroxide), alkali metal hydrides (e.g., sodium hydride, potassium hydride), basic salts (e.g., sodium carbonate, potassium carbonate, sodium hydrogen carbonate, potassium hydrogen carbonate) and the like; and organic bases such as tertiary amines (e.g., triethylamine, diisopropylethylamine, dimethylaminopyridine, 1,8-diazabicyclo[5.4.0]undec-7-ene, 1,5-diazabicyclo[4.3.0]non-5-ene), aromatic amines (e.g., pyridine) and the like. Of these, triethylamine is preferable.

The amount of the base to be used is generally 1 to 10 equivalents, preferably 1 to 5 equivalents, more preferably 2 to 5 equivalents, relative to compound (1).

The halogenation and sulfonylation are preferably carried out in a solvent. The solvent is not particularly limited as long as it does not inhibit the reaction. Specific examples thereof include nitrile solvents such as acetonitrile, propionitrile and the like; ether solvents such as diethyl ether, diisopropyl ether, tert-butyl methyl ether, tetrahydrofuran, 1,4-dioxane, 1,2-dimethoxyethane and the like; halogenated hydrocarbon solvents such as dichloromethane, chloroform, 1,2-dichloroethane, carbon tetrachloride and the like; aromatic solvents such as benzene, toluene and the like; aliphatic hydrocarbon solvents such as hexane, pentane and the like, and the like. Of these, nitrile solvent is preferable, and acetonitrile is particularly preferably. These solvents may be used alone or in a mixture of two or more kinds thereof at an appropriate ratio.

The amount of the solvent to be used is generally 2- to 100-fold weight, preferably 5- to 10-fold weight, relative to compound (1).

The reaction temperature is generally within the range of room temperature to 100°C, preferably within the range of 60 to 80°C.

The reaction time is generally 0.25 to 15 hr, preferably 0.5 to 2 hr.

A method of isolating the obtained compound (2) after completion of the reaction is not particularly limited, and a conventional method is employed. Generally, compound (2) can be isolated by adding water to the reaction mixture and collecting the precipitated crystals by filtration. It is also possible to concentrate or cool the reaction solution, add a poor solvent, and the like prior to the addition of water. After isolation, the crystals may be purified by conventional purification methods as necessary, such as recrystallization, column chromatography and the like.

### Step 2-1: Amination

In this step, compound (3) can be produced by reacting compound (2) with compound (d). Where necessary, the reaction is carried out in the presence of a base.

The amount of e compound (d) to be used is generally 1 to 10 equivalents, preferably 1 to 5 equivalents, more preferably 1 to 2 equivalents, relative to compound (2).

Examples of the base include inorganic bases such as alkali metal hydroxides (e.g., lithium hydroxide, sodium hydroxide, potassium hydroxide), alkali metal hydrides (e.g., sodium hydride, potassium hydride), basic salts (e.g., sodium carbonate, potassium carbonate, sodium hydrogen carbonate, potassium hydrogen carbonate) and the like; and organic bases such as tertiary amines (e.g., triethylamine, diisopropylethylamine, dimethylaminopyridine, 1,8-diazabicyclo[5.4.0]undec-7-ene, 1,5-diazabicyclo[4.3.0]non-5-ene), aromatic amines (e.g., pyridine) and the like. Of these, tertiary amine is preferable, and triethylamine is particularly preferable.

The amount of the base to be used is generally 1 to 10 equivalents, preferably 1 to 5 equivalents, more preferably 2 to 4 equivalents, relative to compound (2).

The reaction is preferably carried out in a solvent. The solvent is not particularly limited as long as it does not inhibit the reaction. Specific examples thereof include alcohol solvents such as methanol, ethanol, propanol, isopropanol and the like; ether solvents such as diethyl ether, diisopropyl ether, tert-butyl methyl ether, tetrahydrofuran, 1,4-dioxane, 1,2-dimethoxyethane and the like; halogenated hydrocarbon solvents such as dichloromethane, chloroform, 1,2-dichloroethane, carbon tetrachloride and the like; aromatic solvents such as benzene, toluene and the like; aliphatic hydrocarbon solvents such as hexane, pentane and the like; nitrile solvents such as acetonitrile, propionitrile and the like; amide solvents such as N,N-dimethylformamide, N,N-dimethylacetamide, N-methylpyrrolidone and the like; sulfoxide solvents such as dimethylsulfoxide and the like; ester solvents such as methyl acetate, ethyl acetate and the like, and the like. Of these, alcohol solvents are preferable, and isopropanol is particularly preferable. These solvents may be used alone or in a mixture of two or more kinds thereof at an appropriate ratio.

The amount of the solvent to be used is generally 2- to 100-fold weight, preferably 5- to 10-fold weight, relative to compound (2).

The reaction temperature is generally within the range of 40 to 120°C, preferably within the range of 50 to 100°C.

The reaction time is generally 10 to 48 hr, preferably 18 to 36 hr.

A method of isolating the obtained compound (3) after completion of the reaction is not particularly limited, and a conventional method is employed. Generally, compound (3) can be isolated by adding water to the reaction mixture and collecting the precipitated crystals by filtration. It is also possible to concentrate or cool the reaction solution, add a poor solvent, and the like prior to the addition of water. After isolation, the crystals may be purified by conventional purification methods as necessary, such as recrystallization, column chromatography and the like.

### Step 2-2: Amination and cyclization

In this step, compound (6) can be produced by reacting compound (2-c) with ammonia. Compound (2-c) is compound (2) wherein P¹ is a hydrogen atom, and P² is a urethane-type protecting group.

The amount of the ammonia to be used is generally 1 to 100 equivalents, preferably 5 to 50 equivalents, more preferably 10 to 30 equivalents, relative to compound (2-c).

The reaction is preferably carried out in a solvent. The solvent is not particularly limited as long as it does not inhibit the reaction. Specific examples thereof include alcohol solvents such as methanol, ethanol, propanol, isopropanol and the like; ether solvents such as diethyl ether, diisopropyl ether, tert-butyl methyl ether, tetrahydrofuran, 1,4-dioxane, 1,2-dimethoxyethane and the like; halogenated hydrocarbon solvents such as dichloromethane, chloroform, 1,2-dichloroethane, carbon tetrachloride and the like; aromatic solvents such as benzene, toluene and the like; aliphatic hydrocarbon solvents such as hexane, pentane and the like; nitrile solvents such as acetonitrile, propionitrile and the like; amide solvents such as N,N-dimethylformamide, N,N-dimethylacetamide, N-methylpyrrolidone and the like; sulfoxide solvents such as dimethylsulfoxide and the like; ester solvents such as methyl acetate, ethyl acetate and the like, and the like. Of these, alcohol solvents are preferable, and isopropanol is particularly preferable. These solvents may be used alone or in a mixture of two or more kinds thereof at an appropriate ratio.

The amount of the solvent to be used is generally 2- to 100-fold weight, preferably 5- to 10-fold weight, relative to compound (2-c).

The reaction temperature is generally within the range of 50 to 200°C, preferably within the range of 70 to 150°C.

The reaction time is generally 12 to 72 hr, preferably 12 to 48 hr.

This reaction may be carried out under pressurized conditions such as in a sealed tube and the like, as necessary.

A method of isolating the obtained compound (6) after completion of the reaction is not particularly limited, and a conventional method is employed. Generally, compound (6) can be isolated by adding water to the reaction mixture, removing the precipitate by filtration and concentrating the filtrate. After isolation, the residue may be purified by conventional purification methods as necessary, such as recrystallization, column chromatography and the like.

### Step 3-1: Deprotection

In this step, compound (4) can be produced by deprotecting the amino group at the 5-position of compound (3-a). Compound (3-a) is compound (3) other than a compound wherein P¹ is a hydrogen atom, and P² is a urethane-type protecting group.

The deprotection of the amino group is determined depending on the kind of the protecting group, and is carried out according to a method known by those of ordinary skill in the art.

When the amino-protecting group is an amide-type protecting group (e.g., acetyl, phenylacetyl), the reaction is carried out, for example, under an acidic condition or basic condition, preferably an acidic condition. Examples of the acidic condition include, conditions using an organic acid such as acetic acid, trifluoroacetic acid and the like; and conditions using a mineral acid such as hydrochloric acid, sulfuric acid, nitric acid and the like. Of these, a condition using hydrochloric acid is preferable.

The amount of the acid to be used is generally 0.05 to 50 equivalents, preferably 0.1 to 10 equivalents, more preferably 0.1 to 5 equivalents, relative to compound (3-a).

The reaction can be carried out in a solvent. However, the reaction is preferably carried out without solvent (the above-mentioned acid is used as a solvent).

When amino-protecting group is a urethane-type protecting group (e.g., benzyloxycarbonyl) or a benzyl group optionally having substituent(s), the reaction is carried out, for example, by catalytic hydrogenation under hydrogen atmosphere.

Examples of the catalyst include palladium/carbon, Raney-nickel alloy, platinum dioxide and the like. Of these, palladium/carbon is preferable.

The amount of the catalyst to be used is preferably 0.005 to 1 equivalent, more preferably 0.01 to 0.2 equivalent, relative to compound (3-a).

The reaction is preferably carried out in a solvent. The solvent is not particularly limited as long as it does not inhibit the reaction. Specific examples thereof include alcohol solvents such as methanol, ethanol, propanol, isopropanol and the like; ether solvents such as diethyl ether, diisopropyl ether, tert-butyl methyl ether, tetrahydrofuran, 1,4-dioxane, 1,2-dimethoxyethane and the like; halogenated hydrocarbon solvents such as dichloromethane, chloroform, 1,2-dichloroethane, carbon tetrachloride and the like; aromatic solvents such as benzene, toluene and the like; aliphatic hydrocarbon solvents such as hexane, pentane and the like; nitrile solvents such as acetonitrile, propionitrile and the like; amide solvents such as N,N-dimethylformamide, N,N-dimethylacetamide, N-methylpyrrolidone and the like; sulfoxide solvents such as dimethylsulfoxide and the like; ester solvents such as methyl acetate, ethyl acetate and the like, and the like. Of these, alcohol solvents are preferable. These solvents may be used alone or in a mixture of two or more kinds thereof at an appropriate ratio.

The amount of the solvent to be used is generally 2- to 100-fold weight, preferably 5- to 10-fold weight, relative to compound (3-a).

The reaction temperature is generally within the range of 0 to 150°C, preferably within the range of 20 to 100°C.

The reaction time is generally 1 to 24 hr, preferably 2 to 5 hr.

This reaction may be carried out under pressurized conditions, as necessary.

A method of isolating the obtained compound (4) after completion of the reaction is not particularly limited, and a conventional method is employed. Generally; compound (4) can be isolated by concentrating the reaction solution, adding a poor solvent (e.g., diethyl ether) thereto, and collecting the precipitated crystals by filtration. After isolation, the crystals may be purified by conventional purification methods as necessary, such as recrystallization, column chromatography and the like.

### Step 3-2: Cyclization

In this step, compound (5) can be produced by treating compound (3-b) with a base to cyclize compound (3-b). Compound (3-b) is compound (3) wherein P¹ is a hydrogen atom, and P² is a urethane-type protecting group (provided that R³ is a hydrogen atom, and R⁴ is not a hydrogen atom).

Examples of the base include metal alkoxides such as sodium methoxide, sodium ethoxide, potassium tert-butoxide and the like, and the like. Of these metal alkoxides are preferable, and potassium tert-butoxide is particularly preferable.

The amount of the base to be used is generally 1 to 10 equivalents, preferably 1 to 5 equivalents, more preferably 2 to 4 equivalents, relative to compound (3-b).

The reaction is preferably carried out in a solvent. The solvent is not particularly limited as long as it does not inhibit the reaction. Specific examples thereof include nitrile solvents such as acetonitrile, propionitrile and the like; ether solvents such as diethyl ether, diisopropyl ether, tert-butyl methyl ether, tetrahydrofuran, 1,4-dioxane, 1,2-dimethoxyethane and the like; halogenated hydrocarbon solvents such as dichloromethane, chloroform, 1,2-dichloroethane, carbon tetrachloride and the like; aromatic solvents such as benzene, toluene and the like; aliphatic hydrocarbon solvents such as hexane, pentane and the like, and the like. Of these, nitrile solvents are preferable, and acetonitrile is particularly preferable. These solvents may be used alone or in a mixture of two or more kinds thereof at an appropriate ratio.

The amount of the solvent to be used is generally 2- to 100-fold weight, preferably 5- to 10-fold weight, relative to compound (3-b).

The reaction temperature is generally within the range of 0 to 100°C, preferably within the range of 25 to 60°C.

The reaction time is generally 1 to 24 hr, preferably 5 to 15 hr.

A method of isolating the obtained compound (5) after completion of the reaction is not particularly limited, and a conventional method is employed. Generally, compound (5) can be isolated by acidifying the reaction mixture and collecting the precipitated crystals by filtration. It is also possible to concentrate or cool the reaction solution, add a poor solvent, and the like prior to the acidification. After isolation, the crystals may be purified by conventional purification methods as necessary, such as recrystallization, column chromatography and the like.

The thus-obtained compounds (4) to (6) are useful as intermediates for compounds having various pharmacological activities, such as antiobesity drug, antidepressant and the like. Compound (2) and compound (3) which are the intermediates for the production of compounds (4) to (6) are novel compounds.

### Examples

The present invention is explained in more detail in the following by referring to Reference Examples and Examples, which are not to be construed as limitative, and can be modified without substantially departing from the spirit and scope of the present invention.

### Reference Example 1

### 5-benzyloxycarbonylamino-6-oxo-2-phenyl-1,6-dihydropyrimidine

To a solution of methyl N-benzyloxycarbonylglycinate (1.25 g, 5.60 mmol) in toluene (12.5 ml) was added tert-butoxy bisdimethylaminomethane (1.5 ml, 7.28 mmol), and the mixture was stirred at 70°C overnight, washed with saturated brine, and concentrated under reduced pressure. To the residue were added MTBE (13.1 ml) and 1N hydrochloric acid (9.8 ml) under ice-cooling and the mixture was stirred at room temperature for 2 hr. The mixture was partitioned, and the organic layer was washed with saturated brine. A solution (1.08 g) of 28% sodium methoxide in methanol was added dropwise to the organic layer. After completion of the reaction, the mixture was concentrated under reduced pressure. Acetonitrile (18.6 ml) was added to the residue, then benzamidine hydrochloride (0.88 g) was added thereto, and the mixture was stirred overnight at 70°C. The reaction mixture was concentrated and water was added thereto, and the mixture was stirred for 1 hr. The precipitate was filtrated and dried under reduced pressure to give the title compound as crystals (1.30 g, 4.04 mmol).
¹H-NMR(DMSO-d₆) δppm : 5.18(2H, s), 7.32-7.57(8H, m), 8.06-8.09(2H, m), 8.46(1H, s), 8.79(1H, s), 13.0(1H, brs); ¹³C-NMR(DMSO, 400MHz) δ66.1, 124.8, 127.3, 127.6, 127.9, 128.3, 128.6, 131.1, 132.1, 136.3, 150.9, 153.4, 158.8, 158.9; MS(ESI) m/z [MH]⁻ 320.1, m.p. 217.1-218.9°C

### Reference Example 2

### 4-hydroxymethylene-2-methyl-5-oxazolinone sodium salt

To a solution of 4-(N,N-dimethylaminomethylene)-2-methyl-5-oxazolinone (1.7 g, 11 mmol) in acetonitrile (21.2 ml) was added 2N sodium hydroxide solution (6.3 ml) under ice-cooling, and the mixture was stirred overnight at room temperature. Water was evaporated and acetonitrile (1.25 ml) was added thereto. The precipitate was filtered, washed with acetonitrile, and dried under reduced pressure at 100°C for 2 hr to give the title compound as white crystals (1.5 g, 10.06 mmol) .
DSC and TG were measured by a heat analyzer, and an endothermic peak and a clear weight change were not observed up to 220°C. Decomposition occurred at 220°C and above. ¹H-NMR(DMSO-d₆) δppm: 2.00(3H, s), 8.67(3H, s); MS(API-ES) m/z [MH]⁺ 126.1

### Reference Example 3

### 5-acetylamino-6-oxo-2-phenyl-1,6-dihydropyrimidine

To a solution of 4-hydroxymethylene-2-methyl-5-oxazolinone sodium salt (1.5 g, 10 mmol) in acetonitrile (33.5 ml) was added benzamidine hydrochloride (1.58 g), and the mixture was stirred overnight at 80°C. The reaction mixture was concentrated, water was added thereto and the mixture was stirred for 1 hr. The precipitate was collected by filtration and dried under reduced pressure to give the title compound as crystals (1.20 g, 5.24 mmol).
¹H-NMR(DMSO-d₆) δppm : 2.15(3H, s), 7.50-7.58(3H, m), 8.06-8.09(2H, m), 8.85(1H, s), 9.50(1H, s), 13.0(1H, brs); ¹³C-NMR(DMSO, 400MHz) δ23.6, 125.2, 127.2, 128.6, 131.1, 132.0, 169.5, 150.5, 150.6, 169.5; MS(ESI) m/z [MH]⁻ 228.1, m.p. 280.8-281.5°C

### Example 1

### 5-acetylamino-4-chloro-2-phenylpyrimidine

To a solution of 5-acetylamino-6-oxo-2-phenyl-1,6-dihydropyrimidine (0.8 g, 3.5 mmol) in acetonitrile (7.0 ml) were added triethylamine (0.25 ml, 1.75 mmol), tetraethylammonium chloride (1.16 g, 7.0 mmol) and phosphorus oxychloride (1.47 ml, 15.75 mmol), and the mixture was stirred at 80°C for 1 hr. After completion of the reaction, saturated aqueous sodium hydrogen carbonate solution was added under ice-cooling and the mixture was stirred for 30 min. When foaming stopped, water was added thereto, and the precipitate was collected by filtration and dried under reduced pressure to give the title compound as crystals (0.79 g, 3.19 mmol)
(yield 91.1%).
¹H-NMR(DMSO-d₆) δppm : 2.18(3H, s), 7.52-7.56(3H, m), 8.30-8.33(2H, m), 9.19(1H, s), 10.02(1H, s); ¹³C-NMR(DMSO, 400MHz) δ23.1, 127.5, 128.8, 129.1, 131.1, 135.3, 152.5, 153.9, 159.2, 169.3; MS(ESI) m/z [MH]⁻ 246.1, m.p. 179.0-179.9°C

### Example 2

### 5-benzyloxycarbonylamino-4-chloro-2-phenylpyrimidine

To a solution of 5-benzyloxycarbonylamino-6-oxo-2-phenyl-1,6-dihydropyrimidine (1.0 g, 3.11 mmol) in acetonitrile (6.2 ml) were added triethylamine (0.22 ml, 1.56 mmol), tetraethylammonium chloride (1.03 g, 6.22 mmol) and phosphorus oxychloride (1.3 ml, 14.0 mmol), and the mixture was stirred at 80°C for 30 min. After completion of the reaction, a saturated aqueous sodium hydrogen carbonate solution was added in an ice bath, and the mixture was stirred for 30 min. When foaming stopped, water was added, and the precipitate was collected by filtration and dried under reduced pressure to give the title compound as crystals (0.925 g, 2.72 mmol)
(yield 75.7%).
¹H-NMR(DMSO-d₆) δppm : 5.22(2H, s), 7.36-7.56(8H, m), 8.30-8.32(2H, m), 9.10(1H, s), 9.84(1H, s); ¹³C-NMR(DMSO, 400MHz) δ66.7, 127.2, 127.6, 128.0, 128.1, 128.4, 128.8, 131.1, 135.3, 136.0, 153.8, 154.0, 158.8, 159.4; MS(ESI) m/z [MH]⁻ 338.1, m.p. 120.0-121.3°C

### Example 3

### 4-chloro-2-phenyl-5-phenylacetylaminopyrimidine

To a solution of 6-oxo-2-phenyl-5-phenylacetylamino-1,6-dihydropyrimidine (0.18 g, 0.59 mmol) in acetonitrile (1.2 ml) were added triethylamine (0.041 ml, 0.30 mmol), tetraethylammonium chloride (0.20 g, 1.18 mmol) and phosphorus oxychloride (0.25 ml, 2.65 mmol), and the mixture was stirred at 70°C for 1 hr. After completion of the reaction, a saturated aqueous sodium hydrogen carbonate solution was added in an ice bath, and the mixture was stirred for 30 min. When foaming stopped, water was added, and the precipitate was collected by filtration and dried under reduced pressure to give the title compound as crystals (0.175 g, 0.54 mmol)
(yield 91.6%).
¹H-NMR(DMSO-d₆) δppm : 3.83(2H, s), 7.35-7.55(8H, m), 8.30-8.32(2H, m), 9.18(1H, s), 10.20(1H, s); ¹³C-NMR(DMSO, 400MHz) δ42.6, 110.0, 120.0, 127.1, 128.1, 128.7, 129.3, 129.6, 131.6, 135.7, 141.8, 154.4, 159.9, 170.6; MS(ESI) m/z [MH]⁻ 322.1, m.p. 168.6-168.9°C

### Example 4

### 4-chloro-2-methoxy-5-phenylacetylaminopyrimidine

To a solution of 2-methoxy-6-oxo-5-phenylacetylamino-1,6-dihydropyrimidine (50 mg, 0.19 mmol) in acetonitrile (0.39 ml) were added triethylamine (0.014 ml, 0.096 mmol), tetraethylammonium chloride (64 mg, 0.39 mmol) and phosphorus oxychloride (0.081 ml, 0.87 mmol), and the mixture was stirred at 70°C for 30 min. After completion of the reaction, a saturated aqueous sodium hydrogen carbonate solution was added in an ice bath, and the mixture was stirred for 30 min. When foaming stopped, water was added, and the precipitate was collected by filtration and dried under reduced pressure to give the title compound as crystals (36 mg, 0.13 mmol) (yield 80.2%).
¹H-NMR(DMSO-d₆) δppm : 3.72(2H, s), 3.93(3H, s), 7.23-7.35(5H, m), 8.70(1H, s), 10.02(1H, s); ¹³C-NMR(DMSO, 400MHz) δ41.9, 55.4, 124.5, 126.5, 128.2, 129.1, 135.3, 155.9, 157.7, 161.6, 169.9; MS(ESI) m/z [MH]⁻ 276.2

### Example 5

### 4-chloro-2-methylthio-5-phenylacetylaminopyrimidine

To a solution of 2-methylthio-6-oxo-5-phenylacetylamino-1,6-dihydropyrimidine (50 mg, 0.18 mmol) in acetonitrile (0.36 ml) were added triethylamine (0.013 ml, 0.091 mmol), tetraethylammonium chloride (60 mg, 0.36 mmol) and phosphorus oxychloride (0.076 ml, 0.82 mmol), and the mixture was stirred at 75°C for 30 min. After completion of the reaction, a saturated aqueous sodium hydrogen carbonate solution was added in an ice bath, and the mixture was stirred for 30 min. When foaming stopped, water was added, and the precipitate was collected by filtration and dried under reduced pressure to give the title compound as crystals (48 mg, 0.16 mmol) (yield 89.8%).
¹H-NMR(DMSO-d₆) δppm : 2.53(3H, s), 3.76(2H, s), 7.25-7.35(5H, m), 8.82(1H, s), 10.09(1H, s); ¹³C-NMR(DMSO, 400MHz) δ14.3, 42.4, 126.8, 127.0, 128.7, 129.6, 135.7, 154.3, 155.6, 167.9, 170.4; MS(ESI) m/z [MH]⁻ 292.1

### Example 6

### 5-acetylamino-4-methylamino-2-phenylpyrimidine hydrochloride

To a solution of 5-acetylamino-4-chloro-2-phenylpyrimidine (0.1 g, 0.40 mmol) in isopropyl alcohol (0.8 ml) were added methylamine hydrochloride (0.03 g, 0.44 mmol) and triethylamine (0.13 ml, 0.89 mmol), and the mixture was stirred at 70°C overnight. The reaction mixture was allowed to cool to room temperature, and concentrated under reduced pressure. Acetonitrile was added, water was added in an ice bath, and the precipitate was collected by filtration and dried under reduced pressure to give the title compound as crystals (93 mg, 0.384 mmol) (yield 95.0%).
¹H-NMR(DMSO-d₆) δppm : 2.09(3H, s), 3.04(3H, d), 7.51-7.54(4H, m), 8.28-8.31(3H, m), 9.45(1H, s); ¹³C-NMR(DMSO, 400MHz) δ23.6, 27.5, 116.3, 127.9, 128.0, 128.7, 129.3, 130.4, 138.1, 157.8, 169.8; MS(ESI) m/z [M+Cl]⁻ 277.1, m.p. 167.3-169.0°C

### Example 7

5-acetylamino-4-benzylamino-2-phenylpyrimidine hydrochloride

To a solution of 5-acetylamino-4-chloro-2-phenylpyrimidine (0.1 g, 0.40 mmol) in isopropyl alcohol (0.8ml) were added benzylamine (0.048 g, 0.44 mmol) and triethylamine (0.13 ml, 0.89 mmol), and the mixture was stirred at 70°C overnight. The reaction mixture was allowed to cool to room temperature, and concentrated under reduced pressure. Acetonitrile was added, water was added in an ice bath, and the precipitate was collected by filtration and dried under reduced pressure to give the title compound as crystals (0.11 g, 0.346 mmol) (yield 85.6%).
¹H-NMR(DMSO-d₆) δppm : 2.08(3H, s), 4.73(2H, d), 7.21-7.55(9H, m), 8.24-8.27(3H, m), 9.31 (1H, s); ¹³C-NMR(DMSO, 400MHz) δ23.7, 43.8, 116.3, 127.1, 127.7, 127.8, 128.6, 128.7, 130.2, 138.3, 140.3, 150.6, 157.0, 159.8, 169.7; MS(ESI) m/z [M+Cl]⁻ 353.1, m.p. 161.9-164.5°C

### Example 8

### N-(5-acetylamino-2-phenyl-4-pyrimidinyl)glycine tert-butyl ester hydrochloride

To a solution of 5-acetylamino-4-chloro-2-phenylpyrimidine (0.1 g, 0.40 mmol) in isopropyl alcohol (0.8 ml) were added tert-butylglycine hydrochloride (0.075 g, 0.44 mmol) and triethylamine (0.13 ml, 0.89 mmol), and the mixture was stirred at 70°C for 2 days. The reaction mixture was allowed to cool to room temperature, and concentrated under reduced pressure. Acetonitrile was added, water was added in an ice bath, and the precipitate was collected by filtration and dried under reduced pressure to give the title compound as crystals (0.11 g, 0.326 mmol) (yield 80.7%).
¹H-NMR(DMSO-d₆) δppm : 1.40(9H, s), 2.50 (3H, s), 4.05(2H, d), 7.42-7.46(4H, m), 8.30-8.32(3H, m), 9.37(1H, s); ¹³C-NMR(DMSO, 400MHz) δ23.6, 28.1, 43.7, 80.8, 116.5, 127.8, 128.6, 137.9, 150.4, 156.7, 159.3, 169.6, 169.7; MS(ESI) m/z [M+Cl]-377.1, m.p. 80.4-85.3°C

### Example 9

### 5-acetylamino-4-amino-2-phenylpyrimidine

To 5-acetylamino-4-chloro-2-phenylpyrimidine (0.2 g, 0.81 mmol) was added a 2M ammonia-isopropyl alcohol solution (10 ml), and the mixture was stirred at 100°C in an autoclave for 3 days. The reaction mixture was allowed to cool to room temperature, and concentrated under reduced pressure. Acetonitrile was added, water was added in an ice bath, and the precipitate was collected by filtration and dried under reduced pressure to give the title compound as crystals (0.13 g, 0.57 mmol) (yield 70.7%).
¹H-NMR(DMSO-d₆) δppm : 2.07(3H, s), 6.82(2H, brs), 7.44-7.47(3H, m), 8.26-8.28 (2H, m), 8.36(1H, s), 9.22(1H, s); ¹³C-NMR(DMSO, 400MHz) δ23.7, 115.8, 127.7, 128.6, 130.1, 138.1, 150.6, 158.4, 159.8, 169.5; MS(ESI) m/z [M+Cl]⁻227.1, m.p. 223.5-224.7°C

### Example 10

### N-(5-benzyloxycarbonylamino-2-phenyl-4-pyrimidinyl)glycine-tert-butyl ester hydrochloride

To a solution of 5-benzyloxycarbonylamino-4-chloro-2-phenylpyrimidine (0.25 g, 0.74 mmol) in isopropyl alcohol (1.5 ml) were added tert-butylglycine hydrochloride (0.14 g, 0.44 mmol) and triethylamine (0.23 ml, 0.89 mmol), and the mixture was stirred at 70°C for 3 days. The reaction mixture was allowed to cool to room temperature, concentrated under reduced pressure and purified by silica gel column chromatography (hexane:ethyl acetate=1:1) to give the title compound (0.27 g, 0.62 mmol) (yield 84.4%).
¹H-NMR(DMSO-d₆) δppm : 1.40(9H, s), 4.07(2H, s), 5.17(2H, s), 7.32-7.55(10H, m), 8.31-8.39(3H, m), 9.06(1H, brs); ¹³C-NMR(DMSO, 400MHz) δ27.7, 43.3, 66.3, 80.4, 116.2, 127.4, 127.6, 127.9, 128.1, 128.4, 130.0, 136.3, 137.3, 148.7, 154.2, 155.8, 158.5, 169.3; MS(ESI) m/z [M+Cl]⁻ 469.1

### Example 11

### 4-methylamino-5-phenylacetylamino-2-phenylpyrimidine hydrochloride

To a solution of 4-chloro-2-phenyl-5-phenylacetylaminopyrimidine (55 mg, 0.17 mmol) in isopropyl alcohol (0.34 ml) were added methylamine hydrochloride (13 mg, 0.187 mmol) and triethylamine (0.052 ml, 0.37 mmol), and the mixture was stirred at 70°C overnight. The mixture was allowed to cool to room temperature, and the precipitate was collected by filtration and dried under reduced pressure to give the title compound as crystals (46 mg, 0.145 mmol) (yield 85.1%).
¹H-NMR(DMSO-d₆) δppm : 3.14(3H, d), 3.84(2H, s), 7.26-7.67(9H, m), 8.25-8.27(2H, d), 8.56(1H, s), 10.25(1H, brs); ¹³C-NMR(DMSO, 400MHz) δ27.6, 42.8, 116.3, 126.9, 127.9, 128.6, 129.7, 130.3, 136.1, 138.1, 149.4, 157.5, 159.7, 170.5; MS(ESI) m/z [M+Cl]-353.1

### Example 12

### 4-benzylamino-5-phenylacetylamino-2-phenylpyrimidine hydrochloride

To a solution of 4-chloro-2-phenyl-5-phenylacetylaminopyrimidine (30 mg, 0.093 mmol) in isopropyl alcohol (0.24 ml) were added benzylamine (11 mg, 0.10 mmol) and triethylamine (0.028 ml, 0.20 mmol), and the mixture was stirred at 80°C overnight. The reaction mixture was allowed to cool to room temperature, concentrated under reduced pressure and purified by silica gel column chromatography (hexane:ethyl acetate=1:2) to give the title compound as crystals (20.4 mg, 0.052 mmol) (yield 55.8%).
¹H-NMR(DMSO-d₆) δppm : 3.14(3H, d), 3.84(2H, s), 7.26-7.67(9H, m), 8.25-8.27(2H, d), 8.56(1H, s), 10.25(1H, brs); MS(ESI) m/z [M+Cl]⁻ 353.1

### Example 13

### N-(2-phenyl-5-phenylacetylamino-4-pyrimidinyl)glycine tert-butyl ester hydrochloride

To a solution of 4-chloro-2-phenyl-5-phenylacetylaminopyrimidine (55 mg, 0.17 mmol) in isopropyl alcohol (0.34 ml) were added tert-butylglycine hydrochloride (35 mg, 0.21 mmol) and triethylamine (0.057 ml, 0.41 mmol), and the mixture was stirred at 70°C for 2 days. The reaction mixture was allowed to cool to room temperature, and concentrated under reduced pressure. Acetonitrile was added, water was added in an ice bath, and the precipitate was collected by filtration and dried under reduced pressure to give the title compound as crystals (57 mg, 0.136 mmol) (yield 80.2%).
¹H-NMR(DMSO-d₆) δppm : 1.40(9H, s), 3.73(2H, s), 4.09(2H, d), 7.25-7.46(9H, m), 8.30-8.32(2H, m), 8.37(1H, s), 9.59(1H, s); ¹³C-NMR(DMSO, 400MHz) δ28.1, 42.8, 43.8, 80.9, 116.5, 126.9, 127.8, 128.6, 129.7, 130.4, 136.1, 137.9, 150.1, 156.4, 159.3, 169.9, 170.4; MS(ESI) m/z [M+Cl]⁻ 453.1

### Example 14

### 7,9-dihydro-2-phenyl-8H-purin-8-one hydrochloride

To 5-benzyloxycarbonylamino-4-chloro-2-phenylpyrimidine (0.1 g, 0.30 mmol) was added a 2M ammonia-isopropyl alcohol solution (10 ml), and the mixture was stirred at 100°C overnight in an autoclave. The reaction mixture was allowed to cool to room temperature, and concentrated under reduced pressure. Acetonitrile was added, water was added in an ice bath, and the precipitate was filtered off. The filtrate was concentrated under reduced pressure, and the residue was purified by silica gel column chromatography (hexane:ethyl acetate=1:2) to give the title compound as crystals (24 mg, 0.097 mmol) (yield 32.4%).
¹H-NMR(DMSO-d₆) δppm : 6.63(2H, s), 7.46-7.53(3H, m), 8.25-8.28(2H, m), 8.47(1H, s), 9.50(1H, s); ¹³C-NMR(DMSO, 400MHz) δ127.0, 128.7, 130.4, 131.0, 135.6, 148.0, 148.8, 155.0, 156.1; MS(ESI) m/z [M+Cl] 247.1

### Example 15

### 4,5-diamino-2-phenylpyrimidine hydrochloride

To 5-acetylamino-4-amino-2-phenylpyrimidine (20 mg, 0.088 mmol) was added 2N hydrochloric acid (5 ml), and the mixture was stirred under reflux for 5 hr. The reaction mixture was allowed to cool to room temperature, and concentrated under reduced pressure. Diethyl ether was added, and the precipitate was collected by filtration and dried under reduced pressure to give the title compound as crystals (19 mg, 0.085 mmol) (yield 96.6%).
¹H-NMR(DMSO-d₆) δppm : 7.56-7.61(4H, m), 8.15-8.17(3H, m), 8.35(2H, brs), 8.82(2H, brs); ¹³C-NMR(DMSO, 400MHz) δ120.1, 127.0, 127.3, 128.9, 130.8, 131.6, 147.5, 155.9

### Example 16

### (8-oxo-2-phenyl-7,8-dihydropurine-9-yl)acetic acid tert-butyl ester

To a solution of N-(5-benzyloxycarbonylamino-2-phenyl-4-pyrimidinyl)glycine-tert-butyl ester hydrochloride (0.1 g, 0.23 mmol) in acetonitrile (5 ml) was added potassium tert-butoxide (52 mg, 0.46 mmol) in an ice bath, and the mixture was stirred at room temperature for 2 hr and at 45°C overnight. The mixture was allowed to cool to room temperature, water was added, and the mixture was concentrated under reduced pressure. Hydrochloric acid was added in an ice bath, and the precipitate was collected by filtration and dried under reduced pressure to give the title compound as crystals (58 mg, 0.178 mmol) (yield 77.3%).
¹H-NMR(DMSO-d₆) δppm: 1.43(9H, s), 4.61(2H, s), 5.16(1H, s), 7.44-7.51(3H, m), 8.31- 8.34(2H, m), 8.40(1H, s); ¹³C-NMR(DMSO, 400MHz) δ27.5, 40.9, 82.0, 120.6, 126.9, 128.4, 129.8, 133.3, 137.3, 150.1, 152.9, 155.9, 166.3; MS(ESI) m/z [MH1]⁻ 325.2

### Reference Example 4

### 4-chloro-2-dimethylamino-5-phenylacetylaminopyrimidine

To a solution of 2-dimethylamino-6-oxo-5-phenylacetylamino-1,6-dihydropyrimidine (39 mg, 0.143 mmol) in acetonitrile (0.30 ml) were added triethylamine (0.010 ml, 0.072 mmol), tetraethylammonium chloride (50 mg, 0.29 mmol) and phosphorus oxychloride (0.060 ml, 0.65 mmol), and the mixture was stirred at 75°C for 3 hr. After completion of the reaction, a saturated aqueous sodium hydrogen carbonate solution was added in an ice bath, and the mixture was stirred for 30 min. When foaming stopped, water was added, and the precipitate was collected by filtration and dried under reduced pressure to give the title compound as crystals (4.5 mg, 0.0155 mmol) (yield 10.8%).
¹H-NMR(DMSO-d₆) δppm : 3.10(6H, s), 3.65(2H, s), 7.24-7.36(8H, m), 8.30(1H, s), 8.70 (1H, s), 9.73(1H); ¹³C-NMR(DMSO, 400MHz) δ37.3, 42.4, 118.3, 126.9, 127.5, 128.7, 129.0, 129.5, 136.1, 157.9, 170.4; MS(ESI) m/z [MH]⁺ 291.0

### Industrial Applicability

According to the present invention, a method of efficiently producing a 4,5-diaminopyrimidine compound and a derivative thereof, i.e., a 8-oxodihydropurine compound, which are useful as intermediates for a compound having various pharmacological activities, such as an antiobesity drug, an antidepressant and the like, by an industrially suitable method can be provided.

## Claims

1. A 5-aminopyrimidine compound represented by the formula (2): 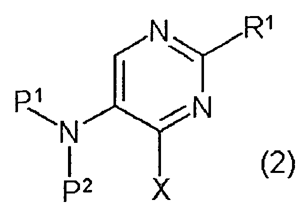 wherein
X is a leaving group,
P¹ is a hydrogen atom or a benzyl group optionally having substituent(s),
P² is a urethane-type protecting group or an amide-type protecting group when P¹ is a hydrogen atom, or a benzyl group optionally having substituent(s) when P¹ is a benzyl group optionally having substituent(s), and
R¹ is a hydrogen atom, an alkyl group optionally having substituent(s), an aryl group optionally having substituent(s), an aralkyl group optionally having substituent(s), or a group represented by the formula (a) or the formula (b):
-O-R² (a)
-S-R² (b)
wherein R² is an alkyl group optionally having substituent(s), an aryl group optionally having substituent(s) or an aralkyl group optionally having substituent(s),
or a salt thereof.

2. A 4,5-diaminopyrimidine compound represented by the formula (3) : 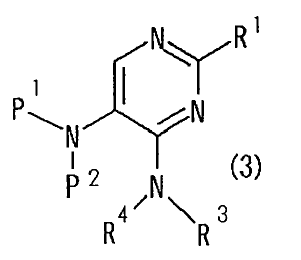 wherein
P¹ is a hydrogen atom or a benzyl group optionally having substituent(s),
P² is a urethane-type protecting group or an amide-type protecting group when P¹ is a hydrogen atom, or a benzyl group optionally having substituent(s) when P¹ is a benzyl group optionally having substituent(s),
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s),
R³ is a hydrogen atom, an alkyl group optionally having substituent(s) or an aralkyl group optionally having substituent(s), and
R⁴ is a hydrogen atom, an alkyl group optionally having substituent(s), an aralkyl group optionally having substituent(s), or a group represented by the formula (c): 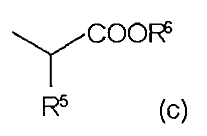 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
provided that a compound wherein P¹ is a hydrogen atom, P² is a urethane-type protecting group and R³ and R⁴ are hydrogen atoms is excluded,
or a salt thereof.

3. A production method of a 5-aminopyrimidine compound represented by the formula (2): 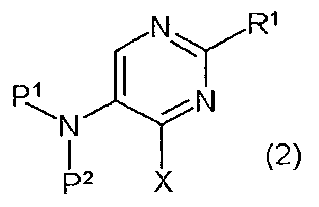 wherein
X is a leaving group,
P¹ is a hydrogen atom or a benzyl group optionally having substituent(s),
P² is a urethane-type protecting group or an amide-type protecting group when P¹ is a hydrogen atom, or a benzyl group optionally having substituent(s) when P¹ is a benzyl group optionally having substituent(s), and
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s),
or a salt thereof, which comprises converting the oxo group of a 5-amino-4-oxopyrimidine compound represented by the formula (1) : 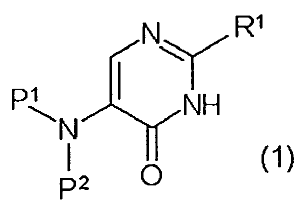 wherein each symbol is as defined above,
or a salt thereof, to a leaving group.

4. A production method of a 4,5-diaminopyrimidine compound represented by the formula (3): 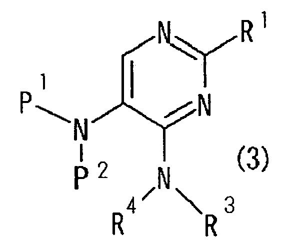 wherein
P¹ is a hydrogen atom or a benzyl group optionally having substituent(s),
P² is a urethane-type protecting group or an amide-type protecting group when P¹ is a hydrogen atom, or a benzyl group optionally having substituent(s) when P¹ is a benzyl group optionally having substituent(s),
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s),
R³ is a hydrogen atom, an alkyl group optionally having substituent(s) or an aralkyl group optionally having substituent(s), and
R⁴ is a hydrogen atom, an alkyl group optionally having substituent(s), an aralkyl group optionally having substituent(s), or a group represented by the formula (c): 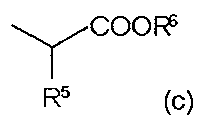 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
or a salt thereof, which comprises reacting a 5-aminopyrimidine compound represented by the formula (2): 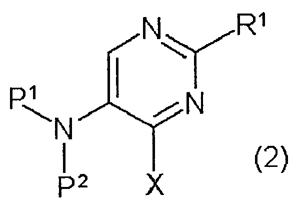 wherein X is a leaving group, and other symbols are as defined above,
or a salt thereof, with an amine compound represented by the formula (d): 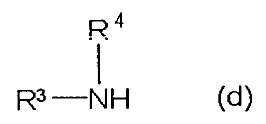 wherein each symbol is as defined above,
or a salt thereof,
provided that when P¹ is a hydrogen atom and P² is a urethane-type protecting group, then both R³ and R⁴ should not be hydrogen atoms.

5. A production method of a 8-oxodihydropurine compound represented by the formula (6): 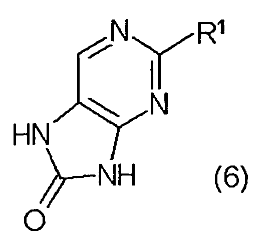 wherein
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s),
or a salt thereof, which comprises reacting a 5-aminopyrimidine compound represented by the formula (2-c): 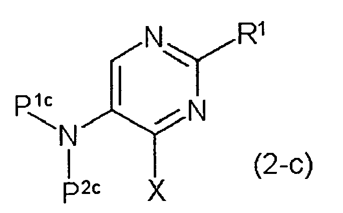 wherein
X is a leaving group,
P^{1c} is a hydrogen atom,
P^{2c} is a urethane-type protecting group, and
R¹ is as defined above,
or a salt thereof with ammonia.

6. A production method of a 4,5-diaminopyrimidine compound represented by the formula (4): 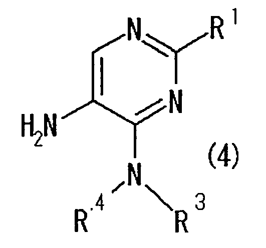 wherein
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s),
R³ is a hydrogen atom, an alkyl group optionally having substituent(s) or an aralkyl group optionally having substituent(s), and
R⁴ is a hydrogen atom, an alkyl group optionally having substituent(s), an aralkyl group optionally having substituent(s), or a group represented by the formula (c): 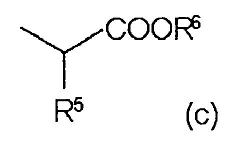 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
or a salt thereof, which comprises removing the amino-protecting group at the 5-position of a 4,5-diaminopyrimidine compound represented by the formula (3-a): 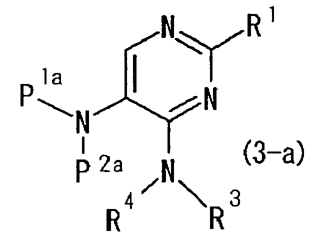 wherein
P^{1a} is a hydrogen atom or a benzyl group optionally having substituent(s),
P^{2a} is an amide-type protecting group when P^{1a} is a hydrogen atom, or a benzyl group optionally having substituent(s) when P^{1a} is a benzyl group optionally having substituent(s), and other symbols are as defined above,
or a salt thereof.

7. A production method of a 8-oxodihydropurine compound represented by the formula (5): 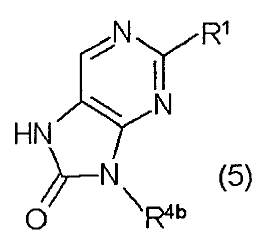 wherein
R¹ is a hydrogen atom, an alkyl group optionally having substituent(s), an aryl group optionally having substituent(s), an aralkyl group optionally having substituent(s), or a group represented by the formula (a) or the formula (b):
-0-R² (a)
-S-R² (b)
wherein R² is an alkyl group optionally having substituent(s), an aryl group optionally having substituent(s) or an aralkyl group optionally having substituent(s), and
R^{4b} is an alkyl group optionally having substituent(s), an aralkyl group optionally having substituent(s), or a group represented by the formula (c): 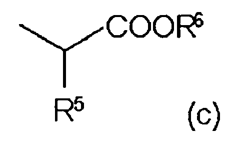 wherein R⁵ is a hydrogen atom, an alkyl group optionally having substituent(s), an aryl group optionally having substituent(s) or an aralkyl group optionally having substituent(s), and R⁶ is an alkyl group optionally having substituent(s) or an aralkyl group optionally having substituent(s),
or a salt thereof, which comprises converting a 4,5-diaminopyrimidine compound represented by the formula (3-b): 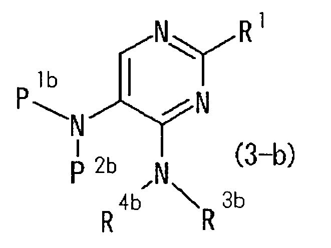 wherein
P^{1b} is a hydrogen atom,
P^{2b} is a urethane-type protecting group,
R^{3b} is a hydrogen atom, and
other symbols are as defined above,
or a salt thereof to a purine derivative.
